(19) Europäisches Patentamt
European Patent Office
Office européen des brevets

(11) Veröffentlichungsnummer: **0 244 398**
**B1**

# (12) EUROPÄISCHE PATENTSCHRIFT

(45) Veröffentlichungstag der Patentschrift: **08.11.89**

(51) Int. Cl.⁴: **C 07 D 498/08**, A 61 K 31/395 // (C07D498/08, 307:00, 267:00)

(21) Anmeldenummer: **85904956.1**

(22) Anmeldetag: **18.10.85**

(86) Internationale Anmeldenummer: **PCT/CH 85/00155**

(87) Internationale Veröffentlichungsnummer: **WO 87/02361 (23.04.87** Gazette **87/9)**

## (54) SUBSTITUIERTE 4-BENZYL-PIPERAZINYL-VERBINDUNGEN.

(43) Veröffentlichungstag der Anmeldung: **11.11.87 Patentblatt 87/46**

(45) Bekanntmachung des Hinweises auf die Patenterteilung: **08.11.89 Patentblatt 89/45**

(84) Benannte Vertragsstaaten: **AT BE CH DE FR GB IT LI LU NL SE**

(56) Entgegenhaltungen: **US-A- 4 005 077**

(73) Patentinhaber: **CIBA-GEIGY AG, Klybeckstrasse 141, CH-4002 Basel (CH)**

(72) Erfinder: **KUMP, Wilhelm, Friedrich-Oser-Strasse 10, CH-4105 Biel-Benken (CH)**

**EP 0 244 398 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind neue Derivate des Rifamycins SV und S mit hoher antibiotischer Wirksamkeit. Es handelt sich um in 3-Stellung durch einen substituierten Piperazin-1-yl-Rest substituierten Rifamycin Verbindungen der Formel

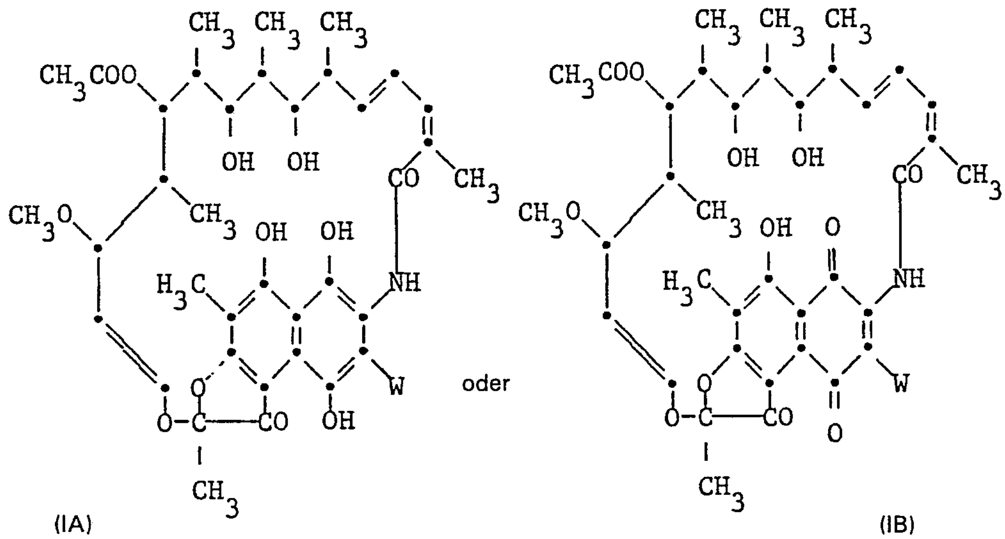

(IA) oder (IB)

worin W einen Piperazin-1-yl-Rest der Formel

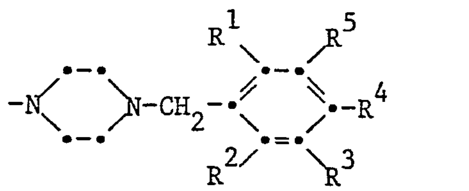

(II)

bedeutet, worin $R^1$ und $R^2$ C(1-4)-Alkyl und $R^3$, $R^4$ und $R^5$ Wasserstoff oder C(1-4)-Alkyl bedeuten, wobei mindestens einer der Reste $R^3$, $R^4$ und $R^5$ von Wasserstoff verschieden ist, oder worin $R^2$ zusammen mit $R^3$ oder $R^3$ zusammen mit $R^4$ gegebenenfalls durch C(1-4)-Alkyl substituiertes Buta-1,3-dien-1,4-ylen, Trimethylen oder Tetramethylen darstellen,

wobei $R^1$, $R^4$ und $R^5$ bzw. $R^1$, $R^2$ und $R^5$ Wasserstoff oder C(1-4)-Alkyl bedeuten, sowie ihre Salze.

Die Erfindung betrifft ferner auch Verfahren zur Herstellung der Verbindungen der Formeln IA und IB sowie ihrer Salze, diese enthaltende pharmazeutische Präparate, sowie die Verwendung dieser Verbindungen und Präparate.

Infolge der sehr engen Beziehung zwischen der 1,4-Chinon- und 1,4-Hydrochinon-Form (entsprechend Rifamycin-S und -SV) und der Leichtigkeit, mit welcher die beiden Formen ineinander übergehen, sind überall, wo nicht spezifisch anders angegeben, beide Formen im Gegenstand der Erfindung inbegriffen, wobei jedoch die SV-Form (IA) als die bevorzugte anzusehen ist.

Die C(1-4)-Alkyle sind z.B. Äthyl, Propyl, i-Propyl, n-Butyl, Isobutyl oder tert.-Butyl, in erster Linie aber Methyl.

Einen bevorzugten Gegenstand der vorliegenden Erfindung stellen die Verbindungen der Formel (IA) und (IB) dar, in welcher W den Rest der Formel II bedeutet, worin $R^1$ und $R^2$ C(1-4)-Alkyl, insbesondere Methyl, $R^4$ C(1-4)-Alkyl, z.B. Methyl oder tert.-Butyl, und $R^3$ und $R^5$ Wasserstoff bedeuten, oder worin $R^2$ und $R^3$ zusammen oder $R^3$ und $R^4$ zusammen Buta-1,3-dien-1,4-ylen und $R^1$, $R^4$ und $R^5$ bzw. $R^1$, $R^2$ und $R^5$ Wasserstoff darstellen, sowie Salze, insbesondere pharmazeutisch verwendbare Salze davon.

In erster Linie betrifft die Erfindung Verbindungen der Formel IA, in welcher der Rest W die Formel II hat, worin $R^1$ und $R^2$ C(1-4)-Alkyl, insbesondere Methyl, $R^4$ C(1-4)-Alkyl, z.B. Methyl oder tert.-Butyl, und $R^3$ und $R^5$ Wasserstoff bedeuten, oder worin insbesondere $R^2$ und $R^3$ zusammen, ferner $R^3$ und $R^4$ zusammen Buta-1,3-dien-1,4-ylen darstellen und $R^1$, $R^4$ und $R^5$ bzw. $R^1$, $R^2$ und $R^5$ Wasserstoff bedeuten, und Salze, insbesondere pharmazeutisch verwendbare Salze davon.

In 4-Stellung des Piperazin-1-yl-Restes substituierte 3-(Piperazin-1-yl)-rifamycine S und SV sind schon beschrieben worden. So werden z.B. im US-Patent 4 005 077 in Spalte 4, Zeilen 3-24, derartige Rifamycin-Derivate erwähnt, worin u.a. in dieser 4-Stellung ein unsubstituierter oder substituierter Kohlenwasserstoffrest vorhanden sein kann, welcher C(1-6)-Niederalkyl oder Mono- oder Dihydroxyniederalkyl oder Niederalkoxy-, Carbalkoxy-, Phenyl- oder Phenyl-niederalkyl sein kann. Unter den Derivaten mit solchen Substituenten werden insbesondere Benzyl- und 1- oder 2-Phenyläthyl-derivate genannt, die im aromatischen Teil durch ein oder mehrere Reste, unter anderem durch Alkyl mit 1-6 C-Atomen, substituiert sein können.

Substituierte 3-(4-Benzyl-piperazin-1-yl)-rifamycine SV und S mit Substituenten der zuletztgenannten Art sind insbesondere im Beispiel 77 des genannten US-Patentes offenbart, so gemäss der in diesem Beispiel erhaltenen Tabelle neben dem 3-(4-Benzyl-piperazin-1-yl)-rifamycin SV das 3-[4-(p-Methylbenzyl)-piperazin-1-yl]-rifamycin SV; 3-[4-(o-Methylbenzyl)-piperazin-1-yl]-rifamycin SV, 3-[4-(m-Methylbenzyl)-piperazin-1-yl]-rifamycin SV, 3-[4-(p-Isopropylbenzyl)-piperazin-1-yl]-rifamycin SV, 3-[4-(2,3-Dimethylbenzyl)-piperazin-1-yl]-rifamycin

SV und 3-[4-(p-tert-Butylbenzyl)-piperazinyl]-rifamycin SV.

Alle diese Verbindungen weisen eine sehr gute antituberkulöse Wirkung auf, wie an Mäusen oder Ratten, die mit Mycobacterium bovis infiziert wurden, gezeigt werden kann. Sie weisen in diesen Tests $ED_{50}$-Werte auf, die ungefähr denjenigen des bekannten antituberkulösen Mittels Rifampicin entsprechen.

Obwohl Rifampicin zu den besten Mitteln für die Behandlung von tuberkulösen Infektionen zählt, ist in manchen Fällen seine relativ kurze Verweildauer im Organismus ein wesentlicher Nachteil. Die Bereitstellung von Wirkstoffen, welche im Vergleich zu Rifampicin eine etwa gleich starke, dabei aber verlängerte Wirkung gegenüber Tuberkulose-Infektionen hätten, ist daher eine der vordringlichsten Aufgaben auf diesem Gebiete. Auch die oben angeführten, im US-Patent 4 005 077 beschriebenen 3-(4-Benzyl-piperazin-1-yl)-rifamycine weisen den gewünschten Vorteil nicht auf: sie übertreffen zwar Rifampicin, wie schon oben erwähnt, in bezug auf antituberkulöse Wirkung in vivo um etwa das Dreifache, sind ihm aber in bezug auf Langzeit-Wirkung kaum überlegen.

Dagegen wurde es nun gefunden, dass sich die neuen Verbindungen gemäss der vorliegenden Erfindung überraschenderweise nicht nur durch eine gute antituberkulöse Wirksamkeit, die etwa derjenigen des Rifampicins entspricht, sondern auch insbesondere durch eine wesentlich erhöhte Verweildauer im Organismus auszeichnen.

Die Unterschiede zwischen den vorbekannten Verbindungen und den neuen Verbindungen der vorliegenden Erfindung können anhand der Datenzusammenstellung der Tabelle I gezeigt werden. Aus der Tabelle geht klar hervor, dass bei Verbindungen A und B die Verweildauer des Wirkstoffs im Organismus wesentlich länger ist als beim Rifampicin oder den Verbindungen 1 bis 4 des US-Patentes 4 005 077. Dies ist besonders klar ersichtlich beim Vergleich der im Benzylkern dreifach methylierten Verbindung A der vorliegenden Erfindung mit den monosubstituierten Analogen des genannten US-Patentes, d.h. den drei Monometylbenzyl-Derivaten (Verbindungen 1 bis 3) einerseits, und einem Benzyl-Derivat mit grösserem Alkylsubstituenten, d.h. dem 3-(4-Isopropyl-piperazin-1-yl)-rifamycin SV (Verbindung 4) andererseits. Eine derartige, in bezug auf die Pharmakokinetik überlegene Wirksamkeit zeigen die erfindungsgemässen Verbindungen A und B aber nicht nur gegenüber den genannten, unter den Allgemeinbegriff 3-(4-Benzyl-piperazin-1-yl)-rifamycin SV und S fallenden Verbindungen, die im US-Patent 4 005 077, Spalte 4, Zeilen 3-24, offenbart sind, sondern auch allgemein gegenüber anderen 3-Piperazinyl-rifamycinen, wie dem 3-(4-Isobutyl-piperazin-1-yl)-rifamycin SV (Verbindung 5) und dem 3-(4-Methyl-piperazin-1-yl)-rifamycin SV (Verbindung 6).

TABELLE I

Antituberkulöse Wirksamkeit und pharmakokinetisches Verhalten der erfindungsgemässen und einigen vorbekannten Wirkstoffe

| 3-(4-R-Piperazin-1-yl)-rifamycin SV | Wirkung gegen Mycobacterium tuberculosis TB $H_3R_v$ | | Pharmakokinetik | | | |
|---|---|---|---|---|---|---|
| | | | Maus | | Ratte | |
| R | MIC (a) (mcg/ml) | $ED_{50}$ p.o. (mg/kg) | t/2 (b) (h) | $C_{max}$ (c) (mcg/ml) | t/2 (b) (h) | $C_{max}$ (c) (mcg/ml) |
| 1. o-Methyl-benzyl | 0,001 | 1,4 | 22,9 | 2,69 | 86 | 1,32 |
| 2. m-Methyl-benzyl | 0,0003 | 1,4 | 11,9 | 3,39 | — | — |
| 3. p-Methyl-benzyl | 0,0001 | 1,2 | 11,5 | 2,28 | — | — |
| 4. p-Isopropyl-benzyl | 0,003 | 1,5 | 20,4 | 1,81 | — | — |
| 5. Isobutyl | 0,0001 | 1,0 | 12,0 | 1,20 | — | — |
| 6. 2-Methyl-allyl | 0,0003 | 1,0 | 21,0 | 1,40 | — | — |
| A. 2,4,6-Trimethyl-benzyl | 0,003 | 4 | 47,4 | 2,95 | ~450 | 1,51 |
| B. 1-Naphthyl-methyl | 0,0003 | 3 | 48,5 | 1,88 | 111 | 1,12 |
| Standard: Rifampicin | 0,003 | 5,9 | 6 | 2,48 | 3,8 | 1,39 |

(a) minimale Hemmkonzentration (1 mcg = $1{,}10^{-5}$ g)
(b) t/2 = Halbwertszeit der Elimination (h = 1 Stunde)
(c) $C_{max}$ = Höchstkonzentration im Plasma (1 mcg = $1{,}10^{-5}$ g)

Die neuen Verbindungen der vorliegenden Erfindung weisen zudem auch eine überraschend hohe Wirksamkeit gegenüber weiteren Mycobacterien auf, insbesondere gegenüber atypischen Mycobacterien welche in letzter Zeit vermehrt als pathogene Mikroorganismen bei AIDS-Kranken aufgefunden und für unmittelbare Todesursache bei diesen Patienten gehalten werden. Anhand Tabelle II wird ge-

zeigt, dass die erfindungsgemässen Verbindungen A und B die von Rifampicin gegenüber einer Reihe von atypischen Mycobacterien aufgewiesene antimikrobielle Wirksamkeit in vitro mehrfach übersteigen. Diese hohe Wirksamkeit ist besonders ausgeprägt in Gruppe c, d.h. bei nonphotochromogenen Mikroorganismen, welche bei AIDS-Erkrankungen zu den gefährlichsten Erregern zählen.

TABELLE II

Wirksamkeit (MIC) gegenüber atypischen Mycobacterien

| Mikroorganismus (Mycobacterium) | MIC m mcg/ml | | |
|---|---|---|---|
| | Verb. A | Verb. B | Rifampicin |
| a) Photochromogene: | | | |
| M. kansasii K 367 | 0,015 | 0,015 | 0,25 |
| b) Scotochromogene: | | | |
| M. scrofulaceum K 1166 | 0,03 | 0,06 | 1 |
| M. xenopei K 716 | 1 | 0,5 | 1 |
| M. aquae K 1165 | 0,03 | 0,03 | 0,25 |
| c) Nonphotochromogene: | | | |
| M. avium K 536 | 0,5 | 1 | 64 |
| M. intracellulare K 181 | 0,03 | 0,125 | 0,5 |
| » K 653 | 0,125 | 0,25 | 2 |
| » K 546 | 0,125 | 1 | 16 |
| » K 550 | 0,25 | 1 | 8 |
| » K 551 | 0,25 | 1 | 1 |

MIC = minimale Hemmkonzentration (1 mcg = $1,10^{-5}$ g)

Die neuen Verbindungen der vorliegenden Erfindung weisen gute antibakterielle Eigenschaften auch gegen andere, insbesondere gram-positive Mikroorganismen auf. So zeigen sie im in vitro-Versuch gegenüber Staphylokokken, wie Staphylococcus aureus K 1098, und gegenüber Streptokokken, wie Streptococcus pyogenes Aronson K 1129 Hemmwirkungen ab etwa 0,005 µg/ml auf. Im in vivo-Versuch, z.B. gegenüber dem obgenannten Staphylococcus, sind die Verbindungen der vorliegenden Erfindung in Dosen ($ED_{50}$) ab etwa 1 mg/kg sowohl bei subkutaner, wie auch oraler Verabreichung wirksam.

Dabei weisen die Verbindungen gemäss der vorliegenden Erfindung eine grosse therapeutische Breite auf, indem sie eine signifikante Toxizität erst bei hohen Dosen, etwa in der Grössenordnung von 5000 mg/kg, aufweisen.

Die neuen Verbindungen können daher als Heilmittel, in erster Linie zur Behandlung von tuberkulösen und AIDS Infektionen, aber auch von anderen Infektionen, wie z.B. Lepra, oder solchen, die durch pyogene Keime, wie z.B. Staphylokokken, hervorgerufen werden, verwendet werden.

Die neuen Verbindungen der Formeln IA und IB können in an sich bekannter Weise hergestellt werden, z.B. indem man

a) ein 3-$R_0$-Rifamycin S, worin $R_0$ für Wasserstoff oder Halogen steht, mit einem Amin der Formel H-W (III) umsetzt, oder

b) ein N'-unsubstituiertes 3-Piperazinylrifamycin S oder -rifamycin SV mit einer Verbindung, die den Rest der Formel

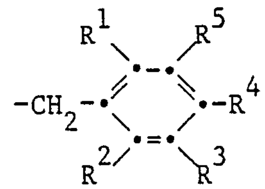

(IV)

in die 4-Stellung des Piperazinrest einzuführen vermag, umsetzt, und, wenn erwünscht, eine erhaltene Verbindung der Formel (IA) und/oder (IB) in eine andere Verbindung der Formel (IA) und/oder (IB) überführt, und/oder ein verfahrensgemäss erhältliches Salz in die freie Verbindung oder in ein anderes Salz und/oder eine verfahrensgemäss erhältliche freie Verbindung in ein Salz überführt.

Die Umsetzung von Rifamycin S ($R_0$ = Wasserstoff) mit dem Amin der Formel III kann in an sich bekannter Weise, wie z.B. in der deutschen Patentschrift 1 670 377 angegeben, durchgeführt werden. So verwendet man zweckmässig einen Überschuss an Amin (etwa 5-10 Mol). Die Reaktion wird z.B. in einem Hydroxygruppen-freien organischen Lösungsmittel, vorzugsweise von geringer Polarität, wie einem halogenierten aliphatischen Kohlenwasserstoff, wie Methylenchlorid oder Chloroform, einem Ester oder Äther, wie z.B. Äthylacetat, Butylacetat, Amylacetat, Cellosolve oder Tetrahydrofuran, und in erster Linie in Dioxan, und vorzugsweise bei Zimmertemperatur oder, z.B. bei langsamem Verlauf, bei erhöhter Temperatur, z.B. zwischen Zimmertemperatur und 100°, durchgeführt. Dabei kann der Verlauf der Reaktion dünnschichtchromatographisch verfolgt werden.

Im allgemeinen bildet sich bei dieser Verfahrensvariante ein Gemisch des erwünschten Reaktionsproduktes in der Chinon- und der Hydrochinonform. Vorzugsweise wird dieses Gemisch, wie unten noch näher beschrieben, vereinheitlicht, indem mittels Reduktion nur die Hydrochinonform (Derivat von Rifamycin SV) oder mittels Oxidation nur die Chinonform (Derivat von Rifamycin S) gebildet wird.

Als 3-Halogen-rifamycin S kann ausser 3-Chlor- und 3-Iod-rifamycin S (vgl. deutsche Patentschrift 2 548 128) insbesondere das 3-Brom-rifamycin S verwendet werden. Der Austausch des Halogenatoms gegen den Rest des Amins der Formel III wird üblicherweise in einem inerten Lösungsmittel, insbesondere in einem Äther, wie Tetrahydrofuran, Dioxan oder in einem halogenierten aliphatischen Kohlenwasserstoff, wie Chloroform, Dichlormethan oder 1,2-Dichloräthan, oder einem aromatischen Kohlenwasserstoff, wie Benzol oder Toluol, vorgenommen, wobei die Reaktion vorzugsweise bei Temperaturen zwischen 0° bis 100° durchgeführt wird; vgl. auch deutsche Offenlegungsschrift 2 847 427.

In der Verfahrensvariante b) verwendet man als Reagens zur Einführung des Restes der Formel IV reaktionsfähige Ester des entsprechenden Alkohols, vor allem Verbindungen der Formel

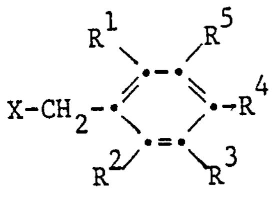

(V)

worin X für den Rest einer starken anorganischen oder organischen Säure steht, wie für den Rest einer Halogen-, wie Chlor-, Brom- oder Iodwasserstoffsäure, einer sauerstoffhaltigen anorganischen Säure, wie Schwefelsäure, Phosphorsäure, phosphorige Säure, Kieselsäure, schweflige Säure, oder einer Halogenschwefelsäure, wie Fluorsulfonsäure, oder einer organischen Sulfonsäure, wie einer aliphatischen oder aromatischen Sulfonsäure, z.B. einer Niederalkansulfonsäure oder einer gegebenenfalls, z.B. durch Niederalkyl oder Nitro; substituierten Benzolsulfonsäure steht. X bedeutet insbesondere Chlor, Brom oder Iod, ferner Methansulfonyloxy oder p-Toluolsulfonyloxy.

Die Umsetzung erfolgt vorzugsweise in Anwesenheit einer Base, insbesondere eines stark basischen, nicht nucleophilen, tertiären Amins, insbesondere eines entsprechenden sterisch gehinderten, aliphatischen und/oder araliphatischen Amins, wie Tri-niederalkyl-amins, z.B. der sogenannten Hünig-Base, d.h. Äthyl-diisopropylamin. Dabei wird die Rifamycin-Verbindung und das Alkylierungsmittel in äquimolaren Mengen verwendet, wobei auch die Base vorzugsweise im äquimolaren Verhältnis zugesetzt wird.

Die Isolierung des Reaktionsproduktes aus einem verfahrensgemäss erhältlichen Reaktionsgemisch erfolgt in an sich bekannter Weise, z.B. durch Verdünnen mit Wasser, und/oder gegebenenfalls durch Neutralisieren mit einer wässrigen Säure, wie einer anorganischen oder organischen Säure, z.B. einer Mineralsäure oder vorteilhaft Zitronensäure, und Zugabe eines mit Wasser nicht mischbaren Lösungsmittels, wie z.B. eines chlorierten Kohlenwasserstoffs, z.B. Chloroform oder Methylenchlorid, wobei das Reaktionsprodukt in die organische Phase übergeht, aus welcher es in üblicher Weise, z.B. durch Trocknen, Eindampfen des Lösungsmittels und Kristallisation und/oder Chromatographie des Rückstandes oder anderen, üblichen Reinigungsmethoden in reiner Form erhalten werden kann.

Die Ausgangsstoffe für die beschriebenen Verfahrensvarianten sind bekannt oder können in an sich bekannter Weise hergestellt werden. So kann z.B. das als Ausgangsstoff zu verwendende 3-Piperazinyl-rifamycin SV nach dem im deutschen Patent 1 676 377 beschriebenen Verfahren aus Rifamycin S und Piperazin mit nachträglicher Reduktion des Produkts mit Ascorbinsäure erhalten werden.

Das Verfahrensprodukt kann in der Hydrochinonform der Formel IA oder in der Chinonform der Formel IB oder, insbesondere das Produkt der Verfahrensmodifikation a), in Form eines Gemisches der beiden erhalten werden. Die beiden Formen können in an sich bekannter Weise ineinander bzw. ein Gemisch der beiden in eine der beiden einheitlichen Formen übergeführt werden. Dabei kann die Umwandlung eines verfahrensgemäss erhältlichen Chinons der Formel IB in das entsprechende Hydrochinon der Formel IA bzw. eines verfahrensgemäss erhältlichen Hydrochinons der Formel IA in ein Chinon der Formel IB, oder die Vereinheitlichung eines Gemisches der beiden Verbindungstypen mittels Reduktion bzw. Oxidation nach oder vorteilhafterweise vor der Isolierung des gewünschten Produkts durchgeführt werden. Die Reduktion kann durch Behandeln mit einem, insbesondere einem zur Reduktion eines Chinons in das entsprechende Hydrochinon geeigneten, Reduktionsmittel, wie einem Alkalimetall-, z.B. Natriumdithionit oder -hydrosulfit, Zink und Essigsäure, oder vorzugsweise mit Ascorbinsäure, die Oxidation durch Behandeln mit einem, insbesondere einem für die Umwandlung eines Hydrochinons in das entsprechende Chinon geeigneten, Oxidationsmittel, wie Luftsauerstoff, Wasserstoffperoxid, Alkalimetall-, z.B. Kalium-, -ferricyanid, einem Persulfat, z.B. Ammoniumpersulfat, oder Mangandioxid bewirkt werden, wobei die Oxidation vorzugsweise unter basischen Bedingungen durchgeführt wird. Die Chinone sind meist violettrot gefärbte Verbindungen, während die Hydrochinone üblicherweise gelbgefärbt sind und besser kristallisieren.

Die Verbindungen der vorliegenden Erfindung können Salze, insbesondere Säureadditionssalze und in erster Linie pharmazeutisch verwendbare Säureadditionssalze mit anorganischen oder organischen Säuren bilden; solche sind u.a. Halogen-, z.B. Chlor- und Bromwasserstoffsäuren, Schwefelsäure, Phosphorsäure, Salpetersäure oder Perchlorsäure, oder aliphatische, alicyclische, aromatische oder heterocyclische Carbon- oder Sulfonsäuren, wie Ameisen-, Essig-, Propion-, Bernstein-, Glykol-, Milch-, Äpfel-, Wein-, Zitronen-, Fumar-, Malein-, Hydroxymalein-, Oxal-, Brenztrauben-, Phenylessig-, Benzoe-, p-Aminobenzoe-, Anthranil-, p-Hydroxybenzoe-, Salicyl-, p-Aminosalicylsäure, Embonsäure, Methansulfon-, Äthansulfon-, Hydroxyäthansulfon-, Äthylendisulfon-, Halogenbenzolsulfon-, Toluolsulfon-, Naphthalinsulfonsäuren oder Sulfanilsäure, ferner Methionin, Tryptophan, Lysin oder Arginin, sowie Ascorbinsäure. Hydrochinonverbindungen vom Typ der Formel IA können auch Salze mit Basen, z.B. Alkalimetall-, wie Natriumsalze bilden.

Die Salze können in an sich bekannter Weise, z.B. durch Behandeln mit einer zur Salzbildung geeigneten Säure oder mit einer Base, z.B. mit der gewünschten Base, wie Ammoniak oder einem organischen Amin, oder einem entsprechenden Metall-, wie Alkalimetallhydroxid, -carbonat oder -hydrogencarbonat, oder mit einem geeigneten Ionenaustauschmittel hergestellt werden.

Verbindungen der vorliegenden Erfindung können auch innere Salze, z.B. durch übliches acido-basisches Titrieren zum Neutralpunkt bzw. zum isoelektrischen Punkt, oder z.B. durch Behandeln mit geeigneten Quaternisierungsmitteln, wie reaktionsfähigen Estern von Niederalkanolen mit starken Säuren,

wie Halogenwasserstoffsäure, Schwefelsäure oder starken organischen Sulfonsäuren, quaternäre Ammoniumsalze bilden.

Diese oder andere Salze der neuen Verbindungen, wie z.B. die Pikrate, können auch zur Reinigung der erhaltenen Verbindungen dienen, indem man die freien Verbindungen in Salze überführt, diese abtrennt und aus den Salzen wiederum die freien Verbindungen gewinnt. Infolge der engen Beziehung zwischen den Verbindungen in freier Form und in Form ihrer Salze sind im vorausgegangenen und nachfolgend unter den freien Verbindungen sinn- und zweckgemäss gegebenenfalls auch die entsprechenden Salze zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen.

Im Hinblick auf die oben beschriebenen pharmakologischen Eigenschaften der neuen Verbindungen umfasst die vorliegende Erfindung auch die Verwendung der erfindungsgemässen Wirkstoffe, allein, z.B. zusammen mit Hilfsstoffen, oder in Kombination mit anderen Wirkstoffen, insbesondere Antibiotika oder Chemotherapeutika, zur Herstellung von Arzneimitteln zur Behandlung von Infektionen, insbesondere solchen, die durch Tuberkellbazillen, atypischen Mycobacterien, insbesondere die von AIDS-Erkrankungen, sowie durch Bakterien und insbesondere Kokken, wie den genannten, hervorgerufen werden, und zwar sowohl als Heilmittel, wie auch als Desinfektionsmittel. Bei der Verwendung werden die erfindungsgemässen Wirkstoffe in therapeutisch wirksamen Mengen, vorzugsweise in Form von pharmazeutischen Präparaten zusammen mit konventionellen pharmazeutischen Trägermaterialien oder Hilfsstoffen verabreicht. Dabei werden z.B. an Warmblüter mit einem Körpergewicht von etwa 70 kg je nach Spezies, Körpergewicht, Alter und individuellem Zustand, sowie je nach Applikationsweise und insbesondere auch je nach der jeweiligen Empfindlichkeit des Krankheitserregers, tägliche Dosen von etwa 50 bis 3000 mg die in akuten Fällen noch mehrfach überschritten werden dürfen, verabreicht.

Die Erfindung betrifft im weiteren pharmazeutische Präparate, welche die Verbindungen der vorliegenden Erfindung als Wirkstoffe enthalten, sowie Verfahren zu ihrer Herstellung.

Bei den erfindungsgemässen pharmazeutischen Präparaten handelt es sich z.B. um solche zur enteralen, wie peroralen oder rektalen, sowie zur parenteralen Verabreichung an Warmblüter. Entsprechende Dosiseinheitsformen, insbesondere zur peroralen Verabreichung, z.B. Dragées, Tabletten oder Kapseln, enthalten vorzugsweise von etwa 50 bis etwa 500 mg, insbesondere von etwa 100 bis etwa 300 mg des Wirkstoffs zusammen mit pharmazeutisch verwendbaren Träger- oder Hilfsstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Tragant, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Überzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann

der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

*Beispiel 1*

Eine Lösung von 5 g 3-Brom-rifamycin S in 50 ml Tetrahydrofuran wird mit 3 g 1-(2,6-Dimethyl-4-tert-butyl-benzyl)-piperazin versetzt und bei 20° während 30 Minuten stehen gelassen. Anschliessend säuert man durch Zugabe von wässriger Zitronensäurelösung an und nimmt das Reaktionsprodukt in Methylenchlorid auf. Nach dem Trocknen und Eindampfen des Methylenchloridextraktes hinterbleibt ein dunkelgefärbter Rückstand; man löst ihn in Methanol und setzt tropfenweise wässrige Ascorbinsäure zu. Das 3-[4-(2,6-Dimethyl-4-tert-butyl-benzyl)-piperazin-1-yl]-rifamycin SV fällt in Form von gelbgefärbten Kristallen aus, Smp. 260°.

*Beispiel 2*

In Analogie zu Beispiel 1 setzt man 3 g 3-Brom-rifamycin S mit 3 g 1-(2,4,6-Trimethyl-benzyl)-piperazin um, und erhält so das 3-[4-(2,4,6-Trimethyl-benzyl)-piperazin-1-yl]-rifamycin SV in gelben Kristallen vom Smp. 178-181° (teilweise unter Zersetzung).

Zur Herstellung des Natriumsalzes dieser Verbindung werden äquivalente Mengen 3-[4-(2,4,6-Trimethyl-benzyl)-piperazin-1-yl]-rifamycin SV und Natriumhydrogencarbonat in einem Gemisch von Dioxan und Wasser gelöst und die Lösung lyophilisiert.

*Beispiel 3*

In Analogie zu Beispiel 1 setzt man 3 g 3-Brom-rifamycin S mit 3 g 1-(1-Naphthylmethyl)-piperazin um, und erhält so das 3-[4-(1-Naphthylmethyl)-piperazin-1-yl]-rifamycin SV in gelben Kristallen, Smp. 177 bis 178°.

Die Herstellung des Natriumsalzes erfolgt in analoger Weise wie in Beispiel 2 beschrieben.

*Beispiel 4*

Kapseln, enthaltend 250 mg 3-[4-(2,4,6-Trimethyl-benzyl)-piperazin-1-yl]-rifamycin SV, können wie folgt hergestellt werden:

*Zusammensetzung* (für 100 Kapseln):

| | |
|---|---|
| 3-[4-(2,4,6-Trimethyl-benzyl)-piperazin-1-yl]-rifamycin SV | 250,0 g |
| Maisstärke | 50,0 g |
| Polyvinylpyrrolidon | 15,0 g |
| Magnesiumstearat | 5,0 g |
| Äthanol | q.s. |

Der Wirkstoff und die Maisstärke werden vermischt und mit einer Lösung des Polyvinylpyrrolidons in 50 g Äthanol befeuchtet. Die feuchte Masse wird durch ein Sieb mit einer Maschenweite von 3 mm gepresst und bei 45° getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Grösse 0 abgefüllt.

**Patentansprüche für die Vertragsstaaten:**
BE, CH, DE, FR, GB, IT, LI, LU, NL, SE

1. Verbindungen der Formeln

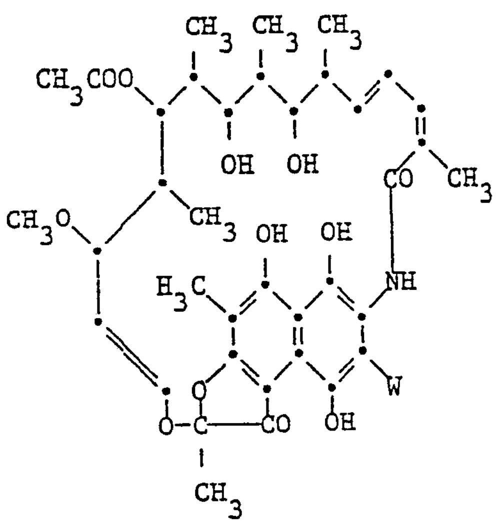

(IA)

oder

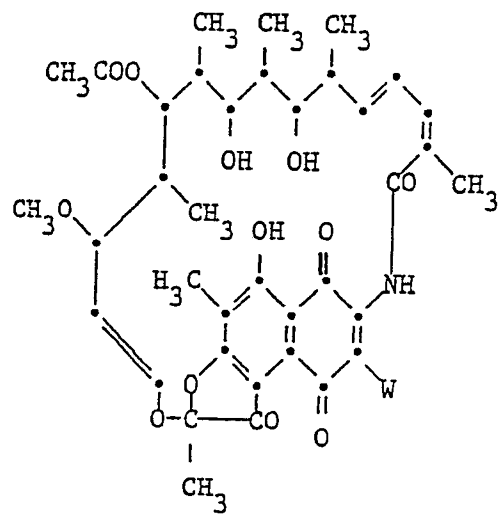

(IB)

worin W einen Piperazin-1-yl-Rest der Formel

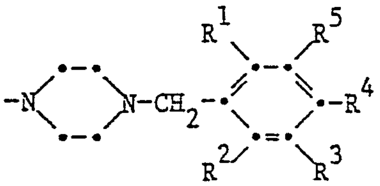

(II)

bedeutet, worin $R^1$ und $R^2$ C(1-4)-Alkyl und $R^3$, $R^4$ und $R^5$ Wasserstoff oder C(1-4)-Alkyl bedeuten, wobei mindestens einer der Reste $R^3$, $R^4$ und $R^5$ von Wasserstoff verschieden ist, oder worin $R^2$ zusammen mit $R^3$ oder $R^3$ zusammen mit $R^4$ gegebenenfalls durch C(1-4)-Alkyl substituiertes Buta-1,3--dien-1,4-ylen darstellen,

wobei $R^1$, $R^4$ und $R^5$ bzw. $R^1$, $R^2$ und $R^5$ Wasserstoff oder C(1-4)-Alkyl bedeuten, sowie ihre Salze.

2. Verbindungen gemäss Anspruch 1 der Formeln (IA) oder (IB), in welchen W den Rest der Formel II bedeutet, worin $R^1$ und $R^2$ C(1-4)-Alkyl, $R^4$ C(1-4)-Alkyl und $R^3$ und $R^5$ Wasserstoff bedeuten, oder worin $R^2$ und $R^3$ zusammen oder $R^3$ und $R^4$ zusammen Buta-1,3-dien-1,4-ylen und $R^1$, $R^4$ und $R^5$ bzw. $R^1$, $R^2$ und $R^5$ Wasserstoff darstellen, sowie Salze davon.

3. Verbindungen gemäss Anspruch 1 der Formel (IA), in welcher der Rest W die Formel II hat, worin $R^1$ und $R^2$ C(1-4)-Alkyl, $R^4$ C(1-4)-Alkyl und $R^3$ und $R^5$ Wasserstoff bedeuten, oder worin $R^2$ und $R^3$ zusammen oder $R^3$ und $R^4$ zusammen Buta-1,3-dien--1,4-ylen darstellen und $R^1$, $R^4$ und $R^5$ bzw. $R^1$, $R^2$ und $R^5$ Wasserstoff bedeuten, und Salze davon.

4. 3-[4-(2,6-Dimethyl-4-tert-butyl-benzyl)-piperazin-1-yl]-rifamycin SV.

5. 3-[4-(2,4,6-Trimethyl-benzyl)-piperazin-1-yl]--rifamycin SV.

6. 3-[4-(1-Naphthylmethyl)-piperazin-1-yl]-rifamycin SV.

7. Die Verbindungen gemäss einem der Ansprüche 1 bis 6 in Form eines pharmazeutisch verwendbaren Salzes davon.

8. Pharmazeutische Zusammensetzungen enthaltend mindestens eine Verbindung der Formel IA oder IB gemäss Anspruch 1, worin W die im Anspruch 1 gegebene Bedeutung hat, oder ein pharmazeutisch verwendbares Salz davon.

9. Verfahren zur Herstellung einer pharmazeutischen Zusammensetzung gemäss Anspruch 8, dadurch gekennzeichnet, dass man den genannten Wirkstoff mit mindestens einem pharmazeutischen Hilfsstoff auf nicht-chemischem Wege verarbeitet.

10. Verwendung von Verbindungen der Formeln IA und IB gemäss Anspruch 1, worin W die im Anspruch 1 gegebene Bedeutung hat, oder pharmazeutisch verwendbaren Salzen davon zur Herstellung von pharmazeutischen Präparaten zur Behandlung von Infektionen, die z.B. durch Mycobacterien und gram-positive Bakterien, wie Staphylokokken, hervorgerufen werden.

11. Verbindungen der Formeln IA und IB gemäss Anspruch 1, worin W die im Anspruch 1 gegebene Bedeutung hat, oder pharmazeutisch verwendbare Salze davon zur Anwendung in einem Verfahren zur therapeutischen Behandlung des menschlichen und tierischen Körpers.

12. Verfahren zur Herstellung der Verbindungen gemäss einem der Ansprüche 1 bis 7, dadurch gekennzeichnet, dass man

a) ein 3-$R_o$-Rifamycin S, worin $R_o$ für Wasserstoff oder Halogen steht, mit einem Amin der Formel H-W (III) umsetzt, oder

b) ein N'-unsubstituiertes-Piperazinylrifamycin S oder -rifamycin SV mit einer Verbindung, die den Rest der Formel

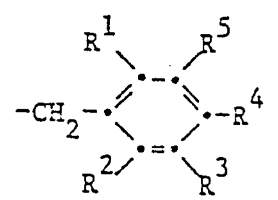

(IV)

in die 4-Stellung des Piperazinrestes einzuführen vermag, umsetzt, und, wenn erwünscht, eine erhaltene Verbindung der Formel (IA) und/oder (IB) in eine andere Verbindung der Formel (IA) und/oder (IB) überführt, und/oder ein verfahrensgemäss erhältliches Salz in die freie Verbindung oder in ein anderes Salz und/oder eine verfahrensgemäss erhältliche freie Verbindung in ein Salz überführt.

**Patentansprüche für den Vertragsstaat:** AT

1. Verfahren zur Herstellung von Verbindungen der Formeln

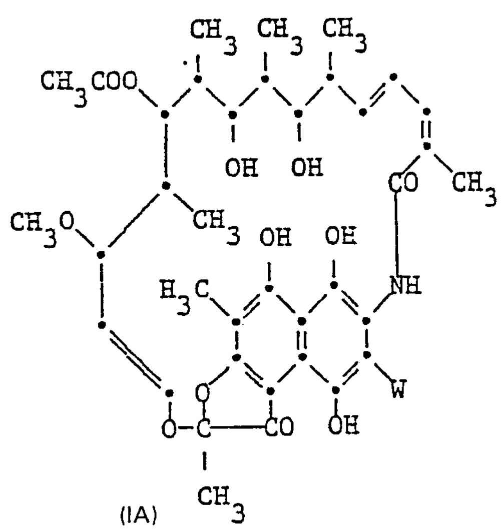

(IA)

oder

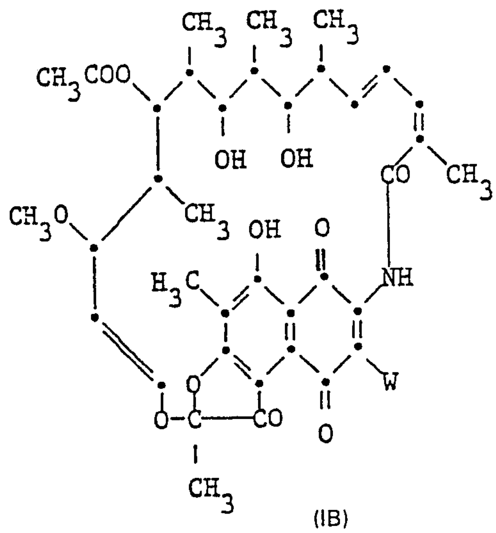

(IB)

worin W einen Piperazin-1-yl-Rest der Formel

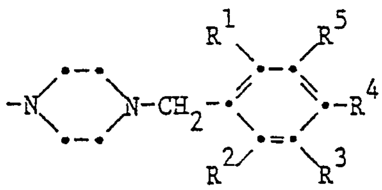

(II)

bedeutet, worin $R^1$ und $R^2$ C(1-4)-Alkyl und $R^3$, $R^4$ und $R^5$ Wasserstoff oder C(1-4)-Alkyl bedeuten, wobei mindestens einer der Reste $R^3$, $R^4$ und $R^5$ von Wasserstoff verschieden ist, oder worin $R^2$ zusammen mit $R^3$ oder $R^3$ zusammen mit $R^4$ gegebenenfalls durch C(1-4)-Alkyl substituiertes Buta-1,3-dien-1,4-ylen darstellen,

wobei $R^1$, $R^4$ und $R^5$ bzw. $R^1$, $R^2$ und $R^5$ Wasserstoff oder C(1-4)-Alkyl bedeuten, sowie ihre Salze, dadurch gekennzeichnet, dass man

a) ein 3-$R_o$-Rifamycin S, worin $R_o$ für Wasserstoff oder Halogen steht, mit einem Amin der Formel H-W (III) umsetzt, oder

b) ein N'-unsubstituiertes-Piperazinylrifamycin S oder -rifamycin SV mit einer Verbindung, die den Rest der Formel

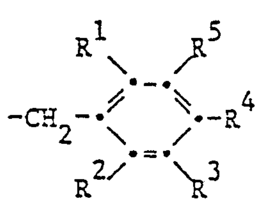

(IV)

in die 4-Stellung des Piperazinrestes einzuführen vermag, umsetzt, und, wenn erwünscht, eine erhaltene Verbindung der Formel (IA) und/oder (IB) in eine andere Verbindung der Formel (IA) und/oder (IB) überführt, und/oder ein verfahrensgemäss erhältliches Salz in die freie Verbindung oder in ein anderes Salz und/oder eine verfahrensgemäss erhältliche freie Verbindung in ein Salz überführt.

2. Verfahren gemäss Anspruch 1 zur Herstellung von Verbindungen der Formeln (IA) oder (IB), in welchen W den Rest der Formel II bedeutet, worin $R^1$ und $R^2$ C(1-4)-Alkyl, $R^4$ C(1-4)-Alkyl und $R^3$ und $R^5$ Wasserstoff bedeuten, oder worin $R^2$ und $R^3$ zusammen oder $R^3$ und $R^4$ zusammen Buta-1,3-dien-1,4-ylen und $R^1$, $R^4$ und $R^5$ bzw. $R^1$, $R^2$ und $R^5$ Wasserstoff darstellen, sowie Salze davon.

3. Verfahren gemäss Anspruch 1 zur Herstellung von Verbindungen der Formel (IA), in welcher der Rest W die Formel II bedeutet, worin $R^1$ und $R^2$ C(1-4)-Alkyl, $R^4$ C(1-4)-Alkyl und $R^3$ und $R^5$ Wasserstoff bedeuten, oder worin $R^2$ und $R^3$ zusammen oder $R^3$ und $R^4$ zusammen Buta-1,3-dien-1,4-ylen darstellen und $R^1$, $R^4$ und $R^5$ bzw. $R^1$, $R^2$ und $R^5$ Wasserstoff bedeuten, und Salze davon.

4. Verfahren gemäss Anspruch 1 zur Herstellung von 3-[4-(2,6-Dimethyl-4-tert-butyl-benzyl)-piperazin-1-yl]-rifamycin SV oder eines Salzes davon.

5. Verfahren gemäss Anspruch 1 zur Herstellung von 3-[4-(2,4,6-Trimethyl-benzyl)-piperazin-1-yl]-rifamycin SV oder eines Salzes davon.

6. Verfahren gemäss Anspruch 1 zur Herstellung von 3-[4-(1-Naphthylmethyl)-piperazin-1-yl]-rifamycin SV oder eines Salzes davon.

7. Verfahren gemäss Anspruch 1 zur Herstellung von Verbindungen gemäss einem der Ansprüche 1 bis 6 in Form eines pharmazeutisch verwendbaren Salzes davon.

8. Verfahren zur Herstellung einer pharmazeutischen Zusammensetzung enthaltend eine Verbindung gemäss einem der Ansprüche 1 bis 6 oder ein pharmazeutisch verwendbares Salz davon, dadurch gekennzeichnet, dass man den genannten Wirkstoff mit mindestens einem pharmazeutischen Hilfsstoff auf nicht-chemischem Wege verarbeitet.

9. Verwendung von Verbindungen der Formeln IA und IB gemäss Anspruch 1, worin W die im Anspruch 1 gegebene Bedeutung hat oder pharmazeutisch verwendbaren Salzen davon zur Herstellung von pharmazeutischen Präparaten zur Behandlung von Infektionen, die z.B. durch Mycobacterien und gram-positive Bakterien, wie Staphylokokken, hervorgerufen werden.

**Claims for the Contracting States:**
BE, CH, DE, FR, GB, IT, LI, LU, NL, SE

1. Compounds of the formula

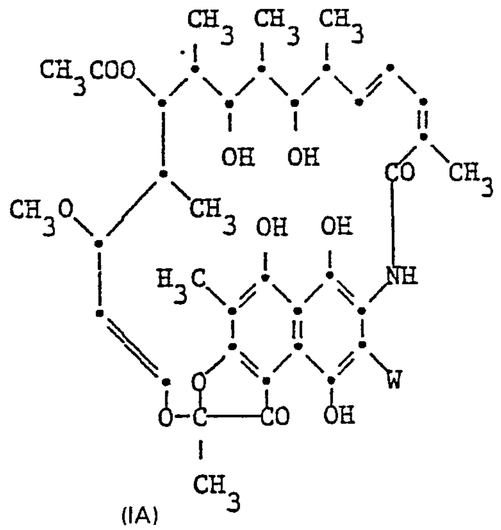

(IA)

or

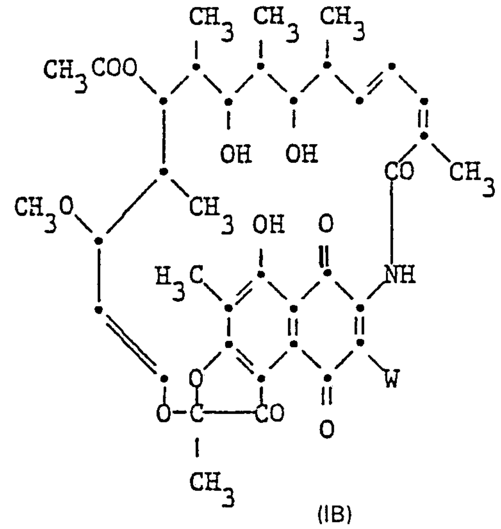

(IB)

wherein W is a piperazin-1-yl radical of the formula

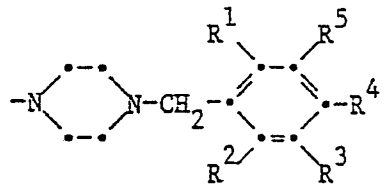

(II)

wherein $R^1$ and $R^2$ are $C_1$-$C_4$alkyl and $R^3$, $R^4$ and $R^5$ are hydrogen or $C_1$-$C_4$alkyl, at least one of the radicals $R^3$, $R^4$ and $R^5$ being other than hydrogen, or $R^2$ together with $R^3$ or $R^3$ together with $R^4$ are buta-1,3-dien-1,4-ylene which is unsubstituted or substituted by $C_1$-$C_4$alkyl, and $R^1$, $R^4$ and $R^5$ or $R^1$, $R^2$ and $R^5$ are hydrogen or $C_1$-$C_4$alkyl, and salts thereof.

2. Compounds according to claim 1 of the formula (IA) or (IB), wherein W is the radical of formula II in which $R^1$ and $R^2$ are $C_1$-$C_4$alkyl, $R^4$ is $C_1$-$C_4$alkyl, and $R^3$ and $R^5$ are hydrogen, or wherein $R^2$ and $R^3$ together or $R^3$ and $R^4$ together are buta-1,3-dien-1,4-ylene, and $R^1$, $R^4$ and $R^5$ or $R^1$, $R^2$ and $R^5$ are hydrogen, and salts thereof.

3. Compounds according to claim 1 of the formula (IA), wherein the radical W has the formula II in which $R^1$ and $R^2$ are $C_1$-$C_4$alkyl, $R^4$ is $C_1$-$C_4$alkyl and $R^3$ and $R^5$ are hydrogen, or wherein $R^2$ and $R^3$ together or $R^3$ and $R^4$ together are buta-1,3-dien-1,4-ylene, and $R^1$, $R^4$ and $R^5$ or $R^1$, $R^2$ and $R^5$ are hydrogen, and salts thereof.

4. 3-[4-(2,6-Dimethyl-4-tert-butylbenzyl)-piperazin-1-yl]-rifamycin SV.

5. 3-[4-(2,4,6-Trimethylbenzyl)-piperazin-1-yl]-rifamycin SV.

6. 3-[4-(1-Naphthylmethyl)-piperazin-1-yl]-rifamycin SV.

7. The compounds as claimed in any one of claims 1 to 6 in the form of a pharmaceutically acceptable salt thereof.

8. Pharmaceutical compositions containing at least one compound of formula IA or IB as claimed in claim 1, wherein W is as defined in claim 1, or a pharmaceutically acceptable salt thereof.

9. A process for the preparation of a pharmaceutical composition as claimed in claim 8, wherein the mentioned active ingredient is processed with at least one pharmaceutical adjuvant by non-chemical means.

10. Use of compounds of the formulae IA and IB according to claim 1, wherein W is as defined in claim 1, or pharmaceutically acceptable salts thereof, for the preparation of pharmaceutical compositions for the treatment of infections caused, for example, by mycobacteria and gram-positive bacteria, such as staphylococci.

11. Compounds of the formulae IA and IB according to claim 1, wherein W is as defined in claim 1, or pharmaceutically acceptable salts thereof, for use in a method for the therapeutic treatment of the human or animal body.

12. A process for the preparation of the compounds according to any one of claims 1 to 7, which comprises

a) reacting a 3-$R_o$-rifamycin S, wherein $R_o$ is hydrogen or halogen, with an amine of formula H-W (III), or

b) reacting a N'-unsubstituted piperazinylrifamycin S or SV with a compound which is capable of introducing the radical of the formula

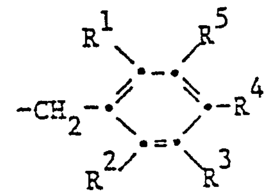

(IV)

into the 4-position of the piperazine radical and, if desired, converting a resultant compound of formula (IA) and/or (IB) into another compound of the formula

(IA) and/or (IB) and/or converting a salt obtainable according to the process into the free compound or into another salt and/or converting a free compound obtainable according to the process into a salt.

**Claims for the Contracting State:** AT

1. A process for the preparation of compounds of the formula

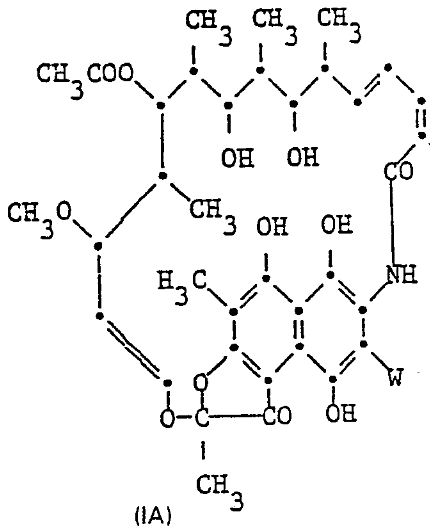

(IA)

or

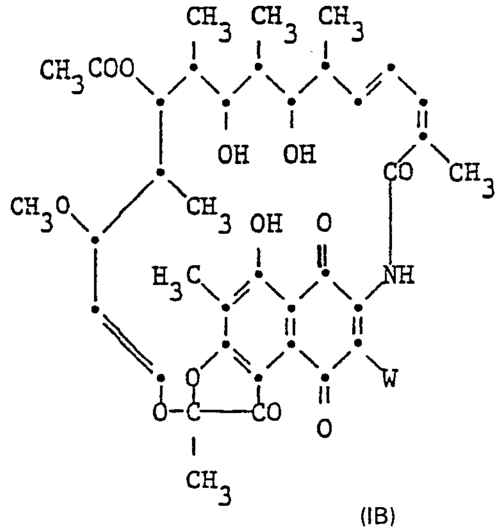

(IB)

wherein W is a piperazin-1-yl radical of the formula

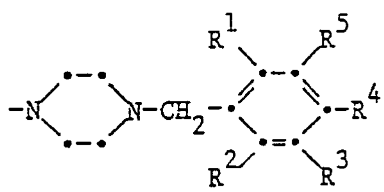

(II)

wherein $R^1$ and $R^2$ are $C_1$-$C_4$alkyl and $R^3$, $R^4$ and $R^5$ are hydrogen or $C_1$-$C_4$alkyl, at least one of the radicals $R^3$, $R^4$ and $R^5$ being other than hydrogen, or $R^2$ together with $R^3$ or $R^3$ together with $R^4$ are buta-1,3-dien-1,4-ylene which is unsubstituted or substituted by $C_1$-$C_4$alkyl, and $R^1$, $R^4$ and $R^5$ or $R^1$, $R^2$ and $R^5$ are hydrogen or $C_1$-$C_4$alkyl, and salts thereof, which process comprises

a) reacting a 3-$R_o$-rifamycin S, wherein $R_o$ is hydrogen or halogen, with an amine of formula H-W (III), or

b) reacting a N'-unsubstituted piperazinylrifamycin S or SV with a compound which is capable of introducing the radical of the formula

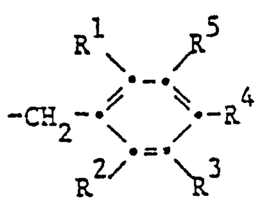

(IV)

into the 4-position of the piperazine radical and, if desired, converting a resultant compound of formula (IA) and/or (IB) into another compound of the formula (IA) and/or (IB) and/or converting a salt obtainable according to the process into the free compound or into another salt and/or converting a free compound obtainable according to the process into a salt.

2. A process according to claim 1 for the preparation of compounds of the formula (IA) or (IB), wherein W is the radical of formula II in which $R^1$ and $R^2$ are $C_1$-$C_4$alkyl, $R^4$ is $C_1$-$C_4$alkyl, and $R^3$ and $R^5$ are hydrogen, or wherein $R^2$ and $R^3$ together or $R^3$ and $R^4$ together are buta-1,3-dien-1,4-ylene, and $R^1$, $R^4$ and $R^5$ or $R^1$, $R^2$ and $R^5$ are hydrogen, and salts thereof.

3. A process according to claim 1 for the preparation of compounds of the formula (IA), wherein the radical W has the formula II in which $R^1$ and $R^2$ are $C_1$-$C_4$alkyl, $R^4$ is $C_1$-$C_4$alkyl and $R^3$ and $R^5$ are hydrogen, or wherein $R^2$ and $R^3$ together or $R^3$ and $R^4$ together are buta-1,3-dien-1,4-ylene, and $R^1$, $R^4$ and $R^5$ or $R^1$, $R^2$ and $R^5$ are hydrogen, and salts thereof.

4. A process according to claim 1 for the preparation of 3-[4-(2,6-dimethyl-4-tert-butylbenzyl)-piperazin-1-yl]-rifamycin SV or a salt thereof.

5. A process according to claim 1 for the preparation of 3-[4-(2,4,6-trimethylbenzyl)-piperazin-1-yl]-rifamycin SV or a salt thereof.

6. A process according to claim 1 for the preparation of 3-{[4-(1-naphthylmethyl)-piperazin-1-yl]-rifamycin SV or a salt thereof.

7. A process according to claim 1 for the preparation of compounds as claimed in any one of claims 1 to 6 in the form of a pharmaceutically acceptable salt thereof.

8. A process for the preparation of a pharmaceutical composition containing a compound as claimed in any one of claims 1 to 6, or a pharmaceutically acceptable salt thereof, wherein the mentioned active ingredient is processed with at least one pharmaceuticall adjuvant by non-chemical means.

9. Use of compounds of the formulae IA and IB according to claim 1, wherein W is as defined in claim 1, or pharmaceutically acceptable salts thereof, for the preparation of pharmaceutical compositions for the treatment of infections caused, for example, by mycobacteria and gram-positive bacteria, such as staphylococci.

**Revendications pour les Etats contractants:**
BE, CH, DE, FR, GB, IT, LI, LU, NL, SE

1. Composé de formules

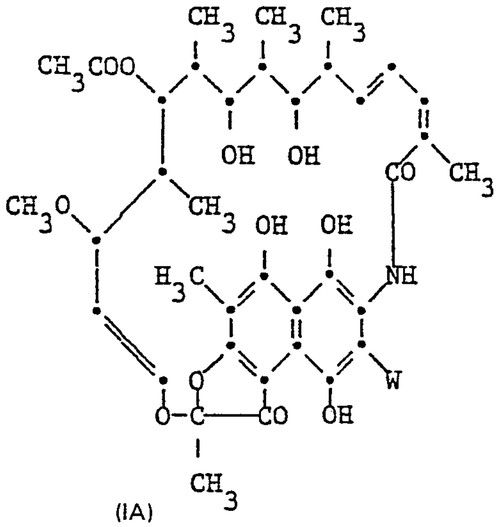

(IA)

ou

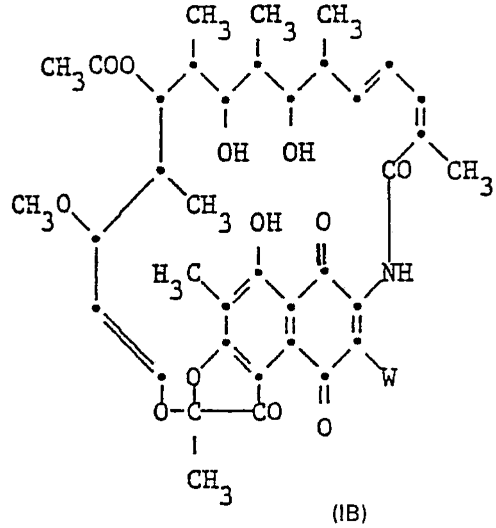

(IB)

dans lesquelles W représente un groupe pipérazine--1-yle de formule

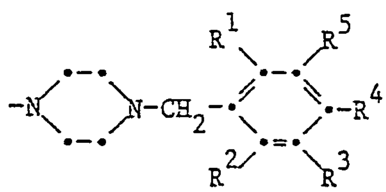

(II)

dans lequel $R^1$ et $R^2$ représentent des groupes alkyle en $C_1$-$C_4$ et $R^3$, $R^4$ et $R^5$ représentent l'hydrogène ou des groupes alkyle en $C_1$-$C_4$, l'un au moins des symboles $R^3$, $R^4$ et $R^5$ ayant une signification autre que l'hydrogène, ou dans lequel $R^2$ forme avec $R^3$ ou $R^3$ avec $R^4$ un groupe buta-1,3-diène-1,4-ylène éventuellement substitué par un groupe alkyle en $C_1$-$C_4$,

auquel cas $R^1$, $R^4$ et $R^5$ ou respectivement $R^1$, $R^2$ et $R^5$ représentent l'hydrogène ou des groupes alkyle en $C_1$-$C_4$, et leurs sels.

2. Composés selon la revendication 1, de formule IA ou IB dans lesquelles W représente le groupe de formule II dans lequel $R^1$ et $R^2$ représentent des groupes alkyle en $C_1$-$C_4$, $R^4$ représente un groupe alkyle en $C_1$-$C_4$ et $R^3$ et $R^5$ représentent l'hydrogène, ou dans lequel $R^2$ et $R^3$, ensemble, ou $R^3$ et $R^4$, ensemble, représentent un groupe buta-1,3-diène--1,4-ylène et $R^1$, $R^4$ et $R^5$ ou respectivement $R^1$, $R^2$ et $R^5$ représentent l'hydrogène, et leurs sels.

3. Composés selon la revendication 1, de formule IA dans laquelle le groupe W répond à la formule II dans laquelle $R^1$ et $R^2$ représentent des groupes alkyle en $C_1$-$C_4$, $R^4$ représente un groupe alkyle $C_1$-$C_4$ et $R^3$ et $R^5$ représentent l'hydrogène, ou dans laquelle $R^2$ et $R^3$, ensemble, ou $R^3$ et $R^4$, ensemble, forment un groupe buta-1,3-diène-1,4-ylène et $R^1$, $R^4$ et $R^5$ ou respectivement $R^1$, $R^2$ et $R^5$ représentent l'hydrogène, et leurs sels.

4. La 3-[4-(2,6-diméthyl-4-tert-butyl-benzyl)-pipérazine-1-yl]-rifamycine SV.

5. La 3-[4-(2,4,6-triméthyl-benzyl)-pipérazine-1--yl]-rifamycine SV.

6. La 3-[4-(1-naphtylméthyl)-pipérazine-1-yl]-rifamycine SV.

7. Les composés selon l'une des revendications 1 à 6, à l'état de sels acceptables pour l'usage pharmaceutique.

8. Compositions pharmaceutiques contenant au moins un composé de formule IA ou IB selon la revendication 1, dans lesquelles W a les significations indiquées dans la revendication 1, ou un sel d'un tel composé acceptable pour l'usage pharmaceutique.

9. Procédé de préparation d'une composition pharmaceutique selon la revendication 8, caractérisé en ce que l'on combine par voie non chimique la substance active en question avec au moins un produit auxiliaire pharmaceutique.

10. Utilisation des composés de formules IA et IB selon la revendication 1, dans lesquelles W a les significations indiquées dans la revendication 1, ou de leurs sels acceptables pour l'usage pharmaceutique, pour la préparation de compositions pharmaceutiques prévues pour le traitement des infections provoquées par exemple par des mycobactéries et des bactéries à gram positif telles que les staphylocoques.

11. Composés de formules IA et IB selon la revendication 1, dans lesquelles W a les significations indiquées dans la revendication 1, ou leurs sels acceptables pour l'usage pharmaceutique, pour l'utilisation dans un procédé pour le traitement thérapeutique de l'organisme humain et animal.

12. Procédé de préparation des composés selon l'une des revendications 1 à 7, caractérisé en ce que:

a) on fait réagir une 3-$R_o$-rifamycine S dans laquelle $R_o$ représente l'hydrogène ou un halogène, avec une amine de formule H-W (III) ou bien

b) on fait réagir une pipérazinyl-rifamycine S ou -rifamycine SV non substitué en N' avec un composé capable d'introduire le groupe de formule

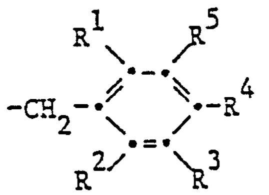

(IV)

dans la position 4 du radical de pipérazine et, si on le désire, on convertit un composé obtenu répondant à la formule IA et/ou IB en un autre composé de formule IA et/ou IB et/ou on convertit un sel obtenu en le composé libre ou en un autre sel et/ou un composé obtenu à l'état libre en un sel.

**Revendications pour l'Etat contractant:** AT

1. Procédé de préparation des composés de formules

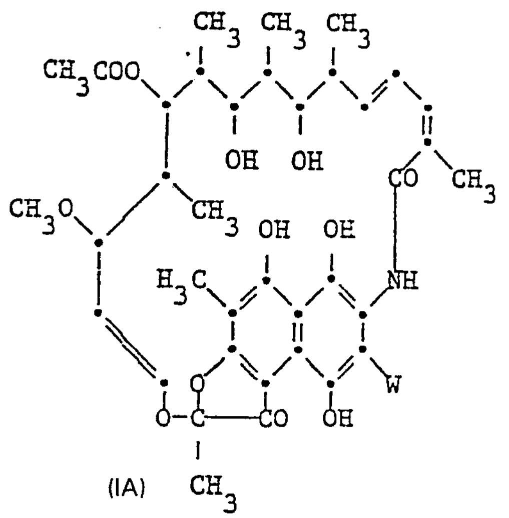

(IA)

ou

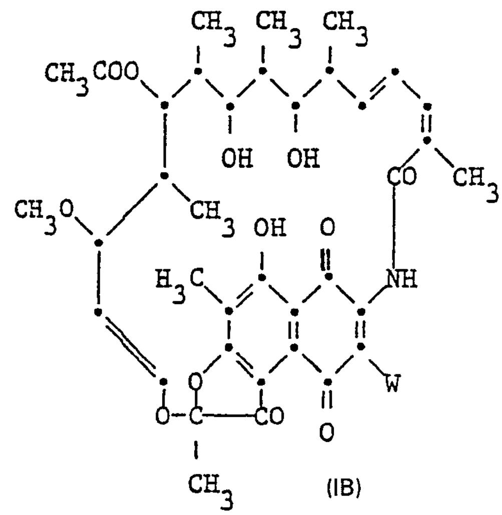

(IB)

dans lesquelles W représente un groupe pipérazine-1-yle de formule

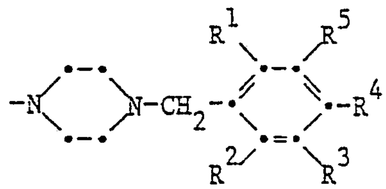

(II)

dans lequel $R^1$ et $R^2$ représentent des groupes alkyle en $C_1$-$C_4$ et $R^3$, $R^4$ et $R^5$ représentent l'hydrogène ou des groupes alkyle en $C_1$-$C_4$, l'un au moins des symboles $R^3$, $R^4$ et $R^5$ ayant une signification autre que l'hydrogène, ou bien dans laquelle $R^2$ forme avec $R^3$ ou $R^3$ avec $R^4$ un groupe buta-1,3-diène-1,4-ylène éventuellement substitué par un groupe alkyle en $C_1$-$C_4$,

auquel cas $R^1$, $R^4$ et $R^5$ ou respectivement $R^1$, $R^2$ et $R^5$ représentent l'hydrogène ou des groupes alkyle en $C_1$-$C_4$, et de leurs sels, caractérisé en ce que:

a) on fait réagir une 3-$R_o$-rifamycine S dans laquelle $R_o$ représente l'hydrogène ou un halogène, avec une amine de formule H-W (III), ou bien

b) on fait réagir une pipérazinyl-rifamycine S ou -rifamycine SV non substituée en N' avec un composé capable d'introduire le groupe de formule

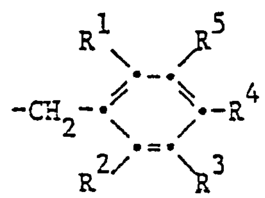

(IV)

dans la position 4 du radical de pipérazine et, si on le désire, on convertit un composé obtenu répondant à la formule IA et/ou IB en un autre composé de formule IA et/ou IB et/ou on convertit un composé obtenu à l'état de sel en le composé libre ou en un autre sel et/ou on convertit un composé obtenu à l'état libre en un sel.

2. Procédé selon la revendication 1, pour préparer les composés de formules IA ou IB dans lesquelles W représente le groupe de formule II dans laquelle $R^1$ et $R^2$ représentent des groupes alkyle en $C_1$-$C_4$, $R^4$ représente un groupe alkyle en $C_1$-$C_4$ et $R^3$ et $R^5$ représentent l'hydrogène, ou bien dans laquelle $R^2$ et $R^3$, ensemble, ou $R^3$ et $R^4$, ensemble, forment un groupe buta-1,3-diène-1,4-ylène et $R^1$, $R^4$ et $R^5$ ou respectivement $R^1$, $R^2$ et $R^5$ représentent l'hydrogène, et leurs sels.

3. Procédé selon la revendication 1, pour la préparation des composés de formule IA dans laquelle W répond à la formule II dans laquelle $R^1$ et $R^2$ représentent des groupes alkyle en $C_1$-$C_4$, $R^4$ représente un groupe alkyle en $C_1$-$C_4$ et $R^3$ et $R^5$ représentent l'hydrogène, ou bien dans laquelle $R^2$ et $R^3$, ensemble, ou $R^3$ et $R^4$, ensemble, forment un groupe buta-1,3-diène-1,4-ylène et $R^1$, $R^4$ et $R^5$ ou respectivement $R^1$, $R^2$ et $R^5$ représentent l'hydrogène, et de leurs sels.

4. Procédé selon la revendication 1, pour préparer la 3-[4-(2,6-diméthyl-4-tert-butyl-benzyl)-pipérazine-1-yl]-rifamycine SV ou l'un de ses sels.

5. Procédé selon la revendication 1, pour préparer la 3-[4-(2,4,6-triméthyl-benzyl)-pipérazine-1-yl]-rifamycine SV ou l'un de ses sels.

6. Procédé selon la revendication 1, pour préparer la 3-[4-(1-naphtylméthyl)-pipérazine-1-yl]-rifamycine SV ou l'un de ses sels.

7. Procédé selon la revendication 1, pour préparer les composés selon l'une des revendications 1 à 6 à

l'état de sels acceptables pour l'usage pharmaceutique.

8. Procédé de préparation d'une composition pharmaceutique contenant un composé selon l'une des revendications 1 à 6 ou un sel acceptable pour l'usage pharmaceutique d'un tel composé, caractérisé en ce que l'on combine par voie non chimique la substance active en question avec au moins un produit auxiliaire pharmaceutique.

9. Utilisation des composés de formules IA et IB selon la revendication 1, dans lesquelles W a les significations indiquées dans la revendication 1, ou de leurs sels acceptables pour l'usage pharmaceutique, pour la préparation de compositions pharmaceutiques prévues pour le traitement d'infections provoquées par exemple par des mycobactéries et des bactéries à gram positif telles que les staphylocoques.